(19) Europäisches Patentamt
European Patent Office
Office européen des brevets

(11) **EP 4 114 262 B1**

(12) **EUROPEAN PATENT SPECIFICATION**

(45) Date of publication and mention
of the grant of the patent:
**27.03.2024 Bulletin 2024/13**

(21) Application number: **21702678.0**

(22) Date of filing: **03.02.2021**

(51) International Patent Classification (IPC):
***A61B 5/145*** (2006.01)      ***G01N 33/49*** (2006.01)
***A61B 5/1473*** (2006.01)

(52) Cooperative Patent Classification (CPC):
**A61B 5/14542; A61B 5/1473;** A61B 2562/0247;
A61B 2562/168; G01N 33/4925

(86) International application number:
**PCT/EP2021/052580**

(87) International publication number:
**WO 2021/175531 (10.09.2021 Gazette 2021/36)**

(54) **MEMBRANE SEALING FOR A PHYSIOLOGICAL SENSOR**

MEMBRANABDICHTUNG FÜR EINEN PHYSIOLOGISCHEN SENSOR

SCELLEMENT DE MEMBRANE POUR UN CAPTEUR PHYSIOLOGIQUE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **05.03.2020 GB 202003198**

(43) Date of publication of application:
**11.01.2023 Bulletin 2023/02**

(73) Proprietor: **SensoCure AS
3185 Skoppum (NO)**

(72) Inventor: **HANSEN, Stein Ivar
3185 Skoppum (NO)**

(74) Representative: **Onsagers AS
P.O. Box 1813 Vika
0123 Oslo (NO)**

(56) References cited:
WO-A1-2004/054438      WO-A1-2021/076194
US-A- 3 659 586        US-A- 4 005 700
US-A1- 2008 011 615    US-A1- 2008 319 278

**Description**

The present invention relates to a technique for sealing a membrane on a physiological sensor, and particularly where the physiological sensor is for measuring partial pressure of carbon dioxide ($pCO_2$), either in vivo or ex vivo.

Ischemia is a medical term for a shortage of blood supply to an organ. If severe, it can lead to death of the affected tissue (infarction). A sensor can be provided to measure tissue $pCO_2$, which is a parameter that increases significantly during the early and reversible stages of ischemia. Such a sensor preferably provides the ability to identify the onset of ischemia events through real-time data.

Ischemia is the most prevalent cause of death in the western world. Thus, for example, myocardial infarction, cerebral infarction and other conditions characterised by hypoperfusion to one or more organs are major factors in mortality.

Reperfusion, reversal of ischemia, is frequently possible if an ischemia is detected in time. Thus, early detection of ischemia followed by appropriate chemical treatment (e.g. with an agent such as streptokinase, urokinase or t-PA which serves to lyse thrombi or emboli) or surgical intervention can save the affected organ as well as the patient's life.

While the heart may be monitored continuously for ischemias using an electrocardiograph (ECG), other organs may become severely ischemic and incur irreversible damage before any symptom is detected. Indeed many organs are "silent" when it comes to ischemia. The phenomenon of silent myocardial infarction is now well recognised. Furthermore, liver and kidney may be severely ischemic without alerting symptoms before the organ damage is irreversible.

It is known that there is a distinct correlation between $pCO_2$ in or on the surface of an organ and the presence of an ischemia in that organ. During tissue metabolic acidosis, e.g. during the anaerobic metabolism that occurs in an ischemia in any organ or tissue, large quantities of carbon dioxide are formed. $CO_2$ is in practical terms freely cell-membrane permeable and since in the ischemia blood flow to transport away the $CO_2$ is absent or restricted, $CO_2$ build up in the ischemic tissue will occur and $pCO_2$ in or on the ischemic tissue will increase.

Generally, in the healthy body, the maximum $pCO_2$ in blood (venous blood) is 6-7 kPa and the maximum $pCO_2$ in healthy (aerobic) tissue is some 1-3 kPa higher, although the maxima may vary from organ to organ, e.g. 8-10 kPa for kidney, 7-10 kPa for liver, and 8-12 kPa for intestines. Where oxygen supply falls below the critical oxygen delivery level, $pCO_2$ values measured in the tissue may rise by 3 to 10 times and the elevated $pCO_2$ levels give a clear indication of anaerobic metabolism and hence, if appropriate, of ischemia.

A sensor particularly suitable for $pCO_2$ measurements is described in WO 2006/08505. The sensor comprises a substantially cylindrical plastic support surrounded by a membrane that is at least partially permeable by carbon dioxide. The support has a conical tip at its distal end and a body portion which extends proximally from the tip. On the body portion are mounted two gold electrodes that extend longitudinally along opposed sides of the body portion. Two frustoconical projections are provided at the proximal and distal ends of the body portion for securing the membrane by frictional fit, optionally supplemented by a crimped connection. The sensor thus defines a closed chamber bounded by membrane and contains the two electrodes. Within the chamber is provided a film of substantially electrolyte-free liquid, such as de-ionised water, that contacts the membrane and both electrodes, so that carbon dioxide crossing the membrane increases the concentration of bicarbonate ions in, and hence the conductivity of, the liquid.

US 2008/319278 describes a physiological sensor for $pCO_2$ measurements wherein a semi-permeable membrane is attached to a support by means of gluing, a crimp connection and a soft gasket, a heat shrink sleeve, or metal crimp rings.

US 3,659,586 describes a physiological sensor for $CO_2$ measurements, wherein a rubber band or O-ring serves to hold the edges of a membrane tensioned over a flange.

US 4,005,700 describes a physiological sensor for $CO_2$ measurement, wherein a membrane is retained by an annular cap which is snap fit over a retaining bead of a support body.

WO 2004/054438 relates to the use of wound filaments to form a mesh structure around a portion of a $pCO_2$ pressure sensor.

EP 4045901A1 relates to the use of a winding filament to secure a gas-permeable membrane to a sensor body of a physiological sensor for $CO_2$ measurement; the teaching of this document is relevant for assessing novelty only.

It has been found that a frictional fit does not secure the membrane sufficiently well, and that a crimped connection undesirably increases the diameter of the sensor. There is thus a need for a new sensor which can be used to determine $pCO_2$.

Viewed from a first aspect, the present invention provides a physiological sensor for measurement of carbon dioxide, comprising: a closed chamber containing a sensor liquid and being bounded, at least partially, by a carbon dioxide permeable membrane; at least two electrodes provided within the chamber in contact with the sensor liquid; a support structure for supporting the membrane; and at least one filament wound at least twice around the support structure and on top of the membrane for securing the gas-permeable membrane to the support structure.

The use of a filament to secure the gas-permeable membrane allows for a gentle, even tension to be applied to the membrane at all points around the sensor, whilst still allowing for small imperfections in the support structure or membrane.

Preferably, the at least one filament exerts a radial force at each point it contacts the membrane, but each wrap can be disconnected from previous windings, meaning that the force is distributed along the length of the winding, but accumulates with successive windings as a resulting force on the membrane. Thus, the filament may create a hermetic seal between the membrane and the support.

The at least one filament is preferably made from a biocompatible and medically approved material. In various exemplary embodiments, the at least one filament may be formed from any one or more of: low-density polyethylene (LDPE), high-density polyethylene (HDPE), ultra-high-molecular-weight polyethylene (UHMWPE), polytetrafluoroethylene (PTFE), ethylene tetrafluoroethylene (ETFE), fluorinated ethylene propylene (FEP), nylon, aramid, or stainless steel.

The filament may be a single stranded filament or a multi-stranded filament.

The at least one filament may be secured to one or more of: itself, the support structure and the carbon dioxide permeable membrane. The at least one filament may be secured by a fixation medium or by welding of the at least one filament. The fixation medium may comprise one or more of an adhesive, a glue and an epoxy, such as a UV curable epoxy. The welding may be achieved by melting or fusing of an end of the at least one filament, such as by heat, by light or by a chemical reaction.

The at least one filament may comprise a first filament wound around the support structure on a first side of the closed chamber, which may be a proximal side of the closed chamber. Optionally, the at least one filament may comprise a second filament wound around the support structure on a second side of the closed chamber, which may be a distal side of the closed chamber. In the present context, a tip of the sensor may be located at the distal end of the sensor. The tip may be configured for placement, in use, into the tissue of an organ.

The two electrodes may optionally extend between the support structure and the membrane beneath one of the filaments.

In one embodiment, the sensor liquid may comprise a substantially electrolyte-free liquid, such as deionised or distilled water. By substantially electrolyte-free, it is meant that the liquid has an ionic osmolality no greater than that at 37°C of an aqueous 5 mM sodium chloride solution, preferably no more than that of a 500 $\mu$M sodium chloride solution, more especially no more than that of a 10-5 to 10-6 M HCl solution.

The mechanism by which $pCO_2$ is determined using the sensor is straightforward. In a pure protic solvent, e.g. water, the electrical resistance is high because of the paucity of ionic species. Addition of $CO_2$ results in formation (with water) of $H^+$ and $HCO_3^-$ ions and thus a reduction in the electrical resistance. Since the only factor responsible for reduction in resistance in the sensor is $CO_2$ passing through the membrane, the change in resistance enables $pCO_2$ to be measured.

From the equilibrium constant for the $H_2O + CO_2$ to $H^+ + HCO_3^-$ equilibrium, $CO_2$ concentration is equal to $\alpha pCO_2$ (where $\alpha$ at 25°C is 0.310). The electrical conductivity for protons is $G_{H+}$ = 349.8 S.cm$^2$/mol, that for hydroxyls is $G_{OH-}$ =198.3 S.cm$^2$/mol and that for bicarbonate is $G_{HCO3-}$ = 44.5 S.cm$^2$/mol. The concentrations of $H^+$ and OH- vary inversely, and the concentrations of $H^+$ and $HCO_3^-$ are directly proportional to $pCO_2$. The total conductance of the solution is thus effectively proportional to $pCO_2$ since the contribution of OH-is minimal. The conductivity of the solution $G_{solution}$ is thus given by:

$$G_{solution} = \theta_{H+}[H^+]G_{H+} + \theta_{OH-}[OH^-]G_{OH-} + \theta_{HCO3-}[HCO_3^-]G_{HCO3-}$$

where $\theta_{H+}$, $\theta_{OH-}$ and $\theta_{HCO3-}$ are the activity coefficients for the three ionic species.

Table 1 below shows, by way of example, measured $pCO_2$ and pH values and corresponding calculated values for $H^+$, OH- and $HCO_3^-$ concentrations showing the increase of $H^+$ and $HCO_3^-$ with increasing $pCO_2$.

| Sample number | pCO$_2$ (kPa) | pH | [H$^+$] (mmol/l) | [OH$^-$] (mmol/l) | [HCO$_3^-$] (mmol/l) |
|---|---|---|---|---|---|
| 1 | 6.38 | 5.141 | 7.23E-06 | 1.38E-09 | 7.23E-06 |
| 2 | 9.64 | 5.060 | 8.71E-06 | 1.15E-09 | 8.71E-06 |
| 3 | 15.37 | 4.891 | 1.29E-05 | 7.78E-10 | 1.29E-05 |
| 4 | 25.88 | 4.760 | 1.74E-05 | 5.75E-10 | 1.74E-05 |
| 5 | 31.48 | 4.664 | 2.17E-05 | 4.61E-10 | 2.17E-05 |

($pCO_2$ and pH measured with a standard blood gas analyser, AB L(R) System 625 at 37°C).

The sensor liquid may further contain at least one metal or metal or metalloid ion, i.e. the sensor liquid may be an electrolyte solution. It has been found that the addition of at least one metal or metalloid ion to the sensor liquid results in a significant increase in sensitivity to $CO_2$.

The concentration of the metal and/or metalloid ions in the liquid may be in the range 0.01 to 20 mmolL$^{-1}$, preferably 0.5 to 18 mmolL$^{-1}$, more preferably 0.1 to 15 mmolL$^{-1}$, even more preferably 0.25 to 12 mmolL$^{-1}$, such as 0.5 to 10 mmolL$^{-1}$.

Typical metal ions include any transition metal or a metal from Group 1, 2, 13 or 14 in the Periodic Table. It will be understood that the term "metalloid" used herein refers to a chemical element with properties intermediate between those of typical metals and non-metals. Example metalloids include boron, silicon and germanium.

In one preferred embodiment, the metal and metalloid ions are selected from the group consisting of: transition metals, Li, Na, Be, Mg, B, Al, Ga, In, Tl, Nh, Si, Ge, Sn, Pb and Fl. A particularly preferred group of metal and metalloid ions are: Cr, Mn, Fe, Co, Ni, Cu, Zn, Pd, Ag, Cd, Al, Ga, In and Tl, especially Al, Ni, Ag, Cu, Co and Pd.

Preferably, the metal ions include Cu ions. Where a mixture of metal and/or metalloid ions are present, one preferable embodiment is a mixture of Cu and Al ions. Another preferable mixture is Cu, Al and Ni ions.

The metal or metalloid ions may be generated in situ by the addition of an appropriate hydroxide, salt or complex of the required metal or metalloid ion(s). In an alternative embodiment, the metal and/or metalloid ions may be provided as isolated ions added directly to the liquid, typically in solution. In yet another embodiment the metal and/or metalloid ions are generated in situ from metal surfaces or interfaces present in the sensor, for example via chemical or electrochemical reactions involving one or more metals.

In one embodiment, the sensor liquid may comprise a non-ionic excipient. In this way, the osmolality of the sensor liquid in the chamber can be increased to prevent egress of the sensor liquid across the membrane, without affecting the electrical characteristics of the sensor liquid. The excipient should have at least isotonic concentration, i.e. should be as osmotic with an aqueous solution of 0.9% w/v NaCl. Thus, the osmolality of the excipient in the chamber may be greater than that of 0.9% w/v aqueous NaCl, preferably greater than that of 1.8% w/v aqueous NaCl (twice isotonic concentration). Osmolalities greater than that of 4.5% w/v aqueous NaCl (five times isotonic concentration), or even greater than that of 9% w/v aqueous NaCl (ten times isotonic concentration) may be used.

Any suitable non-ionic excipient may be used that is inert to the proton and bicarbonate reaction in the chamber. The excipient should also be soluble in the sensor liquid, for example water. The excipient is also desirably an accepted pharmaceutical excipient for intravenous use and with low viscosity for simple filling of the chamber. The excipient should preferably be sterilizable and storage stable. Desirably, the excipient should inhibit microbiological growth.

A suitable excipient is polyethylene glycol (PEG) and the presently preferred excipient is propylene glycol.

Preferably, the sensor liquid is aqueous and especially preferably it comprises water, substantially electrolyte-free as defined above. Other solvents that react with $CO_2$ to increase or decrease their conductance, e.g. by the production or neutralization of ions, may likewise be used. In practice, however, deionized or distilled water with or without the addition of a strong acid (e.g. HCl) to a concentration of 0.1 to 100 $\mu$M, preferably 0.5 to 50 $\mu$M, more especially about 1 $\mu$M, has been found to function particularly well. The function of this small addition of acid is generally to maintain the pH of the liquid at 6 or below to avoid significant contributions to conductance by hydroxyl ions and to maintain the linearity of the measurements of pCOz.

The $pCO_2$ sensor may function by applying an alternating electrical potential to the electrodes whereby to cause an alternating current in the sensor liquid. The sensor liquid should be reactive with carbon dioxide to alter its conductance. The electrical potential may have a frequency of 20 to 10,000 Hz, preferably 100 to 4,000 Hz.

The $pCO_2$ sensor may be provided with or may be connectable to an electrical power source arranged to apply an alternating electrical potential across the electrodes with a frequency of 100 to 10,000 Hz. The frequency is preferably greater than 1 kHz. The frequency is preferably less than 5 kHz, more preferably less than 2 kHz. At frequencies below 100 Hz, the sensitivity of $pCO_2$ determination is lower due to electropolarization and moreover the instrument response time becomes overly slow, while at frequencies above 10 kHz sensitivity is again less due to the low impedance of the capacitances in the sensor. The power source may be an AC power source or alternatively a DC source in conjunction with an oscillator, i.e. a combination which together constitutes an AC power source. The power supply is preferably such that the maximum current density through the liquid at the electrodes is no more than 50 A/m$^2$, preferably no more than 30 A/m$^2$, more preferably no more than 20 A/m$^2$, in particular no more than 10 A/m$^2$, and most preferably about 1 A/m$^2$ or below. Higher current density values of 20 A/m$^2$ or greater should only be used at the higher frequencies, e.g. 1-10 kHz.

The smallest maximum current density is determined by detection limits, but values down to 10$^{-8}$ A/m$^2$ are usable. The smallest maximum current density however will generally be at least 0.1 $\mu$A/m$^2$.

By operating at such current densities and voltage frequencies, and by appropriate construction, the sensor can determine the conductance/resistance of the liquid into which the $CO_2$ migrates without any significant loss of accuracy arising as a result of the electropolarization of the electrodes.

For particularly high accuracy, the potential or current across the electrodes (and hence the resistance or conductance of the liquid between the electrodes) may be determined using a lock-in amplifier set to the same frequency as that of the voltage generator or electrical power source.

Furthermore it is preferred to incorporate in the detection a high pass filter to screen out current with a frequency less than 100 Hz, preferably less than 150 Hz. The filter is preferably a passive filter, for example a capacitor and a resistor. The power source and the detector circuitry may, if desired, be included in the sensor, in this case, if it is desired that the sensor be wireless, it will preferably also be provided with means enabling the signal to be detected remotely, e.g. a transmitter, for example a RF transmitter. In this way the sensor may be implanted, for example in an at-risk patient. A further electrode may be provided that is electrically connected to the patient, for example to the patient's skin. The signal from this further electrode may be processed with the signal from the sensor in order to compensate for electromagnetic noise from the patient.

Electropolarization effects are considerably reduced by increasing the surface area of the electrodes in contact with the liquid, e.g. by siting the electrodes in wells disposed away from the plane of the membrane or by using non-planar electrode surfaces, e.g. rough or textured surfaces. In general therefore it is desirable to have as large a ratio of surface area of electrode to liquid contact as possible, and as shallow as possible a liquid depth over as much as possible of its area of contact with the membrane. In this way the response time is reduced, electropolarization is reduced, lower frequencies may be used and stray capacitance effects are considerably reduced.

Increased electrical resistance relative to the resistance at the electrodes may be achieved by restricting the cross sectional area of the electrical path through the liquid between the electrodes at a zone in which the liquid is in contact with the membrane, e.g. by decreasing the depth of the liquid for a part of the path between the electrodes, and/or by ensuring a relatively large area of contact between each electrode and the liquid.

The resistance of the sensor liquid at the membrane and between the electrodes may be increased by the use of structural elements to define liquid channels across the membrane between the electrodes, e.g. by disposing the membrane across or adjacent an insulating chamber wall portion in which such channels are formed, for example by etching. Likewise a porous spacer may be disposed between the membrane and the chamber wall to define the depth of the liquid.

Indeed, such spacers are important to use where, under the pressure conditions experienced in use, the membrane is sufficiently flexible and the liquid depth behind the membrane sufficiently small, for the measured conductance to vary with pressure.

The support structure may comprise one or more sealing surfaces. Each filament may be wound about one of the sealing surfaces. Each sealing surface may have a smooth circumferential shape, and is preferably substantially cylindrical.

Optionally, the support structure may comprise a stop on a distal side and/or a proximal side of each sealing surface. The stop may have a circumference that is greater than a circumference of the sealing surface. Thus, the stop may prevent the wound filament from moving along the length of the sensor.

In a preferred arrangement, the support structure may comprise a sensor body having a longitudinal axis. The at least two electrodes may be spaced apart in a direction transverse to the longitudinal axis of the sensor body. The sensor body may comprise a plurality of support members extending outwardly from the longitudinal axis of the sensor body and defining at least one liquid channel between adjacent support members. The at least one liquid channel may provide a fluid pathway between the electrodes. The membrane may be supported by the support members and may provide an outer wall of the liquid channel(s).

This arrangement provides a compact configuration of the sensor with a longitudinal geometry that is suited to insertion in an organ. Furthermore, the support members are able to provide physical support to the membrane, as well as defining liquid channels of small cross-sectional area that allow accurate measurement.

In order to reduce the electropolarisation effect mentioned above, the electrodes may be located in a recess in the sensor body that has a greater cross-sectional area than the liquid channels. In this way, the current density around the electrodes is reduced by the greater volume for liquid.

The electrodes of the sensor may extend longitudinally, for example parallel to the longitudinal axis of the sensor body. Similarly, the liquid channel(s) may be transverse, for example perpendicular, to the longitudinal axis of the sensor body. In a preferred arrangement, the sensor comprises a plurality of liquid channels. For example, the sensor may comprise at least three liquid channels.

The support members may be transverse to the longitudinal axis of the sensor body. For example, the support members may be perpendicular to the longitudinal axis of the sensor body in the circumferential direction. In a preferred arrangement, the support members are in the form of rings formed about the longitudinal axis of the sensor body. The cross-section of the support members may be any suitable shape. For example, the support members may have a rounded, in particular substantially semi-circular cross section, or alternatively may have a substantially triangular, in particular sawtooth, cross-section, or yet further alternatively may have a substantially rectangular cross-section.

The support members may be formed integrally with the sensor body, for example by injection moulding. The sensor preferably comprises at least four support members. The sensor body and/or the sensor may be generally cylindrical. The membrane may be arranged to surround the sensor body.

The described geometry may be applied to any suitable sensor. In the preferred arrangement, the sensor is a $pCO_2$ sensor.

Where the sensor is constructed with the sensor liquid film in place, the electrodes are preferably of, or plated with, an inert material such that the resistivity of the sensor liquid will not change significantly with storage. Suitable materials include platinum (especially black platinum), gold, silver, aluminium and carbon. Gold is particularly preferred. In general inert electrodes which do not generate solvated ions are preferred.

The membrane may be any material which is permeable to $CO_2$, and substantially impermeable to the solvent of the sensor liquid, any electrolyte and water. Polytetrafluoroethylene, e.g. Teflon(R), silicone rubber, polysiloxane, polyolefins or other insulating polymer films may be used, e.g. at thicknesses of 0.5 to 250 $\mu$m.

The thicker the membrane, in general the slower the response time of the sensor will be. However, the thinner the membrane the greater the risk of non-uniformities or of perforation or other damage. Conveniently however the thickness of the membrane will be 1 to 100 $\mu$m, preferably 50 to 100 $\mu$m.

The support of the sensor may be of any suitable material, e.g. plastics. Preferably the material should be capable of withstanding conditions normally used in sterilisation, e.g. radiation sterilization (for example using gamma radiation) or thermal sterilization (for example using temperatures of about 121°C as used in autoclave sterilisation). In the case of thermal sterilization, the liquid will generally be sterile filled into the sensor after sterilization. The walls of the chamber and the membrane may be of the same material, e.g. Teflon®, machined to have self-supporting walls and a thinner gas- permeable membrane. The sensor may be generally relatively inexpensive and so, unlike prior art sensors, may be a single-use device. Moreover the electrode chamber can be made extremely small without difficulty (unlike the prior art glass electrode containing sensors for which miniaturization poses insuperable impedance problems). This arrangement provides a sensor, in particular, a $pCO_2$ sensor, which can be inserted easily into the tissue of an animal, including a human, which can be retained in the tissue during monitoring and which can be removed easily when monitoring is complete.

The device is preferably sufficiently small that it will not cause undue disturbance to the tissue to be monitored. Consequently, the device may have a maximum diameter of 2 mm, preferably 1 mm. The sensors may be readily produced having a size and configuration particularly suited to measuring pCOz on the surface of or in an organ, duct or tissue, e.g. brain, heart, liver, kidney, pancreas, gut, muscle or subcutaneous tissue. This is of particular interest as it allows the functioning of the organ, duct or tissue to be monitored, e.g. during and after transplant, in intensive care, following injury, in the postoperative course, etc. and so allows early detection of ischemia.

The partial pressure determined by the sensor may be a quantified value or it may simply be an indication that $pCO_2$ is above or below one or more threshold values indicative of ischemia or non-ischemia, values which may be varied according to the location of the pCOz measurement site.

The sensor may be used for a single measurement of $pCO_2$ or, more preferably, may be used for continuous or repeated monitoring, especially of an at-risk patient, for example a patient in intensive care, undergoing or recovering from an organ or tissue transplant operation, assessed as having unstable angina, recovering from a coronary artery bypass operation, suffering trauma (e.g. of skeletal muscle), or suffering from hypovolemia (e.g. shock).

The device may comprise a plurality of sensors for respective physiological parameters. For example, the device may comprise an array of sensors. Such sensors may measure one or more of the partial pressure of carbon dioxide, the partial pressure of oxygen, oxygen saturation of haemoglobin, temperature, pH or glucose concentration, for example. In the presently preferred embodiment, the device comprises a temperature sensor and a pCOz sensor.

Viewed from a second aspect, the present invention also provides a method of securing a carbon dioxide permeable membrane of a physiological sensor for measurement of carbon dioxide, the method comprising: applying the carbon dioxide permeable membrane over a support structure so as to define a closed chamber of the physiological sensor; and winding at least one filament at least twice around the support structure on top of the membrane so as to secure the membrane to the support structure.

The physiological sensor may comprise any of the features of the sensor described above, and may include any of the optional features thereof.

The at least one filament may create a hermetic seal between the membrane and the support structure.

The at least one filament is preferably made from a biocompatible and medically approved material. In various exemplary embodiments, the at least one filament may be formed from any one or more of: low-density polyethylene (LDPE), high-density polyethylene (HDPE), ultra-high-molecular-weight polyethylene (UHMWPE), polytetrafluoroethylene (PTFE), ethylene tetrafluoroethylene (ETFE), fluorinated ethylene propylene (FEP), nylon, aramid, or stainless steel.

The filament may be a single stranded filament or a multi-stranded filament.

The method may comprise securing the at least one filament, for example to one or more of: itself, the support structure and the carbon dioxide permeable membrane.

The at least one filament may be secured by using a fixation medium. The fixation medium may comprise one or more of an adhesive, a glue and an epoxy, such as a UV curable epoxy.

The at least one filament may be secured by welding of the at least one filament. The welding may be achieved by melting or fusing of an end of the at least one filament, such as by heat, by light or by a chemical reaction.

The winding may comprise winding a first filament around the support structure on a first side of the closed chamber, which may be a proximal side of the closed chamber. The winding may additionally comprise winding a second filament around the support structure on a second side of the closed chamber, which may be a distal side of the closed chamber.

In one embodiment, the winding may be performed whilst the support structure is submerged in a sensor fluid.

Certain preferred embodiments of the present invention will now be described, by way of example only and with reference to the accompanying drawings, in which:

Figure 1 is a schematic diagram of a sensing system incorporating a $pCO_2$ sensor;
Figure 2 is a schematic diagram illustrating the measurement principle for the $pCO_2$ sensor in the system of Figure 1;
Figure 3 is a partially cutaway view of a first $pCO_2$ sensor;
Figure 4 is a cross-sectional view along line A-A of Figure 3;
Figure 4a is a magnified view of the detail indicated by the circle in Figure 4;
Figure 5 is a view of the first $pCO_2$ sensor with the membrane removed;
Figure 6 illustrates a method of securing the membrane to the first $pCO_2$ sensor;
Figure 7 is a view of a wound filament securing the membrane to the first $pCO_2$ sensor; and
Figures 8 is a perspective view of a second $pCO_2$ sensor.

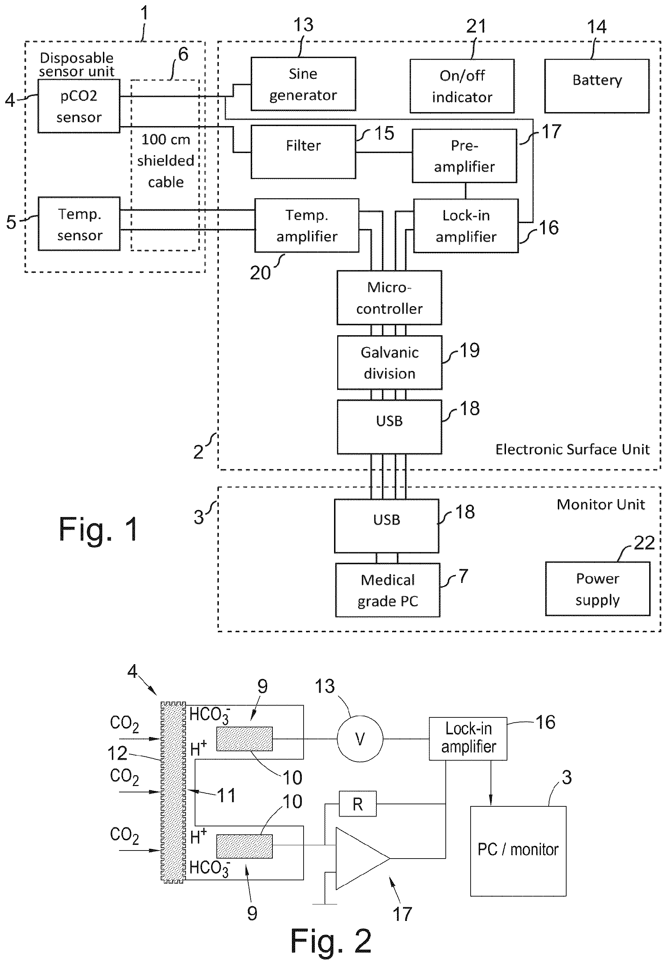

A $pCO_2$ sensing system comprises a disposable sensor unit 1, an electronic surface unit 2, and a monitor unit 3, as shown in Figure 1. The disposable sensor unit 1 is delivered packaged and sterilised. It comprises a membrane-protected conductometric $pCO_2$ sensor 4 with a diameter of less than 1 millimetre, and a temperature sensor 5 integrated in the disposable sensor unit 1. Wires 6 connect the $pCO_2$ sensor 4 and temperature sensor 5 electrically by means of a connector to the electronic surface unit 2.

The electronic surface unit 2 sends and receives signals to and from the sensor unit 1. It performs signal processing and transmits the conditioned signal to the monitor unit 3.

The monitor unit 3 is based on a medical grade PC 7 with a USB interface 18 and Labview software (available from National Instruments Corporation of Austin, Texas).

The $pCO_2$ sensor 4 is used for measurements of the level (partial pressure) of $CO_2$ ($pCO_2$) in a fluid, according to the measurement principle illustrated in Figure 2. A measurement chamber comprises two small cavities 9 with an electrode 10 positioned in each. The two cavities 9 are connected by one or more passageways 11 enclosed by a semi-permeable membrane 12, i.e. a membrane that only allows transport of $CO_2$ in and out of the volume of the sensor 4. The whole volume is filled with a sensor liquid.

The sensor liquid comprises an electrolyte solution or a substantially ion-free liquid, such as deionised water. The sensing liquid may optionally comprise a non-ionic excipient so as to prevent egress of the sensor liquid across the membrane 12, without affecting the electrical characteristics of the sensor liquid. The excipient may comprise, for example between 2% and 40% propylene glycol.

The conductivity in the sensor liquid depends upon the $pCO_2$, and by measuring the conductivity between the electrodes 10 in the volume, information about $pCO_2$ may be extracted.

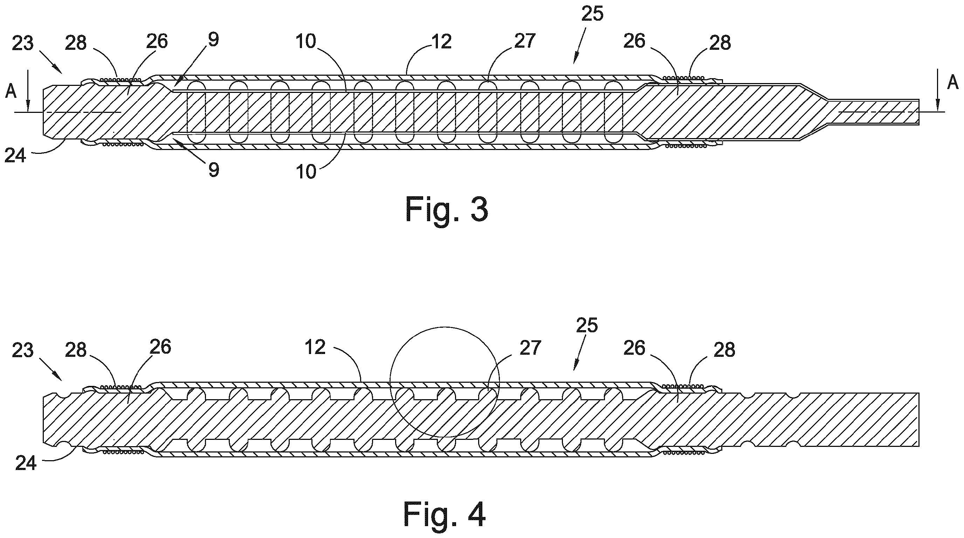

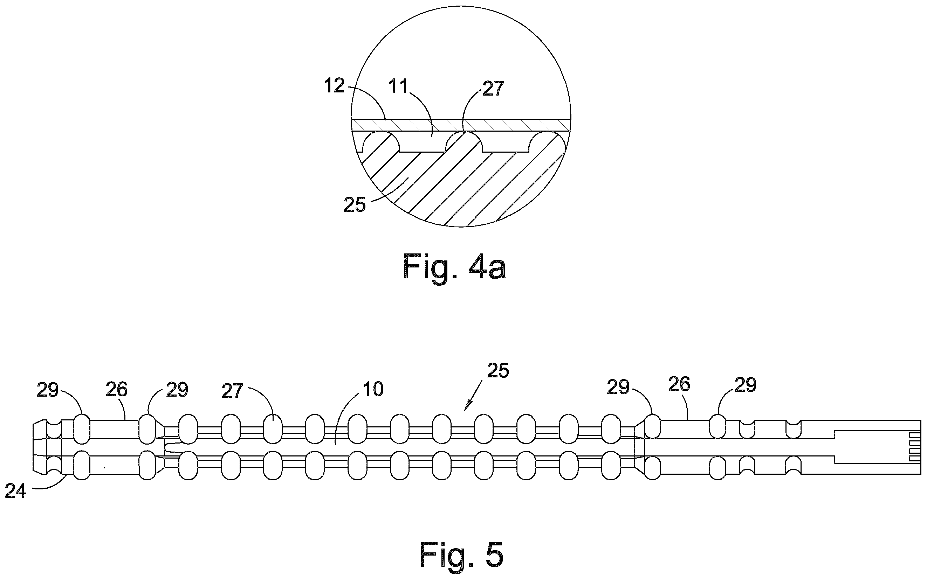

Figures 3 to 7 illustrate a first embodiment of the $pCO_2$ sensor 4. The $pCO_2$ sensor 4 comprises an injection moulded plastics support 23, which is substantially cylindrical and surrounded by the semipermeable membrane 12. The support 23 has a tip 24 at its distal end and a body portion 25 which extends proximally from the tip 24. On the body portion 25 are mounted, such as by gluing, two gold electrodes 10. The electrodes 10 extend longitudinally along opposed sides of the body portion 25 and are received in respective recesses in the body portion 25.

Between the tip 24 and the body portion 25, a sealing surface 26 is provided for securing the membrane 12, as will be discussed in greater detail later. A corresponding sealing surface 26 is provided at the proximal end of the body portion 25. A pair of stop ribs 29 are provided on proximal and distal sides, respectively, of each of the sealing surfaces 26. Furthermore, the sealing surfaces 26 of the support 23 may be made selectively hydrophobic in order to avoid the formation of a water film into which ions may bleed.

The body portion 25 of the support 23 is provided with a plurality of ribs 27, which are formed with a rounded profile. The ribs 27 provide mechanical support to the membrane 12 and also define the fluid passageways 11 required for the $pCO_2$ sensor 4 to function effectively. Between each electrode 10 and the fluid passageways 11 formed between the ribs 27 is provided a reservoir 9 formed by the recess in which the electrode 10 is located. The reservoir 9 provides a region of relatively low current density around the electrodes 10 in order to reduce electropolarisation effects.

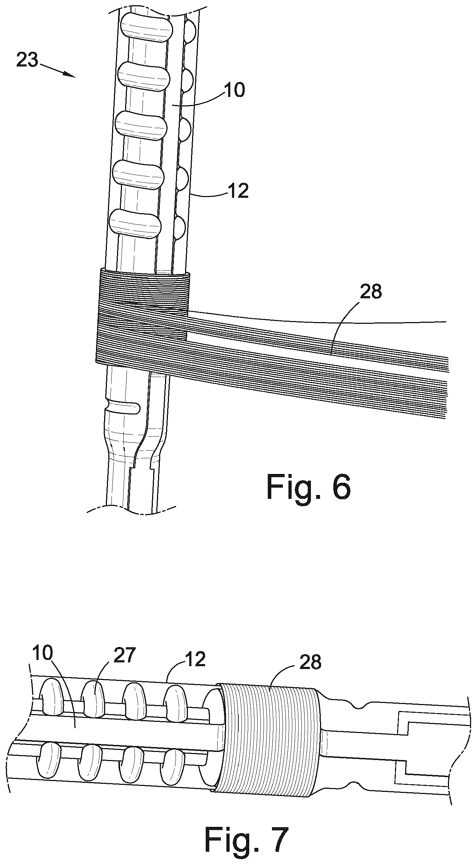

As shown in Figures 6 and 7, during manufacture, the membrane 12 is fixed onto the support 23, while immersed in the sensor liquid, so that the chamber bounded by the membrane 12, the electrodes 10, and the ribs 27 is completely filled with the sensor liquid. Thus, this chamber forms a $pCO_2$ sensor 4 as shown schematically in Figure 2.

Optionally, the membrane 12 can be fixed first onto the support 23 at one end (e.g. the proximal end) whilst out of the sensor liquid. Then, the membrane 12 can be fixed onto the support 23 at the other end (e.g. the distal end) whilst that end is submerged in the sensor liquid. Thus, the liquid filling happens in a separate step in between the two fixing steps.

The membrane 12 is secured to the support 23 using high-strength filament 28 made from a biocompatible and medically approved material, such as used for non-absorbable sutures or ligatures. Exemplary materials may include low-density polyethylene (LDPE), high-density polyethylene (HDPE), ultra-high-molecular-weight polyethylene (UHMWPE), polytetrafluoroethylene (PTFE), ethylene tetrafluoroethylene (ETFE), fluorinated ethylene propylene (FEP), nylon, aramid, etc. Further materials may include organic materials, or metal materials, such as stainless steel. The filament 28 may be single stranded or multi-stranded.

Filament 28 is wound around each of the sealing surfaces 26 of the support 23 on top of membrane 12, such that the filament 28 circumscribes the central axis of the $pCO_2$ sensor 4. This compresses the membrane 12 against the respective sealing surfaces 26 and allows for hermetically sealed closure between membrane 12 and support 23. Winding of the filament can start and/or stop on the support 23 or on the membrane 12. The stop ribs 29 on either side of the sealing surfaces 26 have a greater circumference than the sealing surfaces 26 and act to prevent proximal or distal movement of the membrane 12 where it is compressed by the wound filament 28 with respect to the support 23.

After completion of winding, the wound filament 28 may be covered in a suitable fixation medium, such as an adhesive, glue or epoxy, a UV curable epoxy, etc. Fixation can also be achieved by melting, fusing etc. of the end of the filament 28 to itself or to the membrane 12 or the support 23 by heat, light, chemical reaction etc.

Fixation or fusing of the filament 28 can also take place during winding by the same means (heating, light, chemicals) e.g. by friction.

Using a filament 28 wound on top of the membrane 12 around the $pCO_2$ sensor 4, allows for a gentle, even tension to be applied to the membrane 12 at all points around the $pCO_2$ sensor 4, whilst still allowing for small imperfections in the support 23 or membrane 12.

The filament 28 exerts a radial force at each point it contacts the membrane 12, but each wrap is disconnected from previous windings, meaning that the force is distributed along the length of the winding, but accumulates with increased windings as a resulting force on the membrane 12, creating a hermetical seal between the membrane 12 and the support 23.

The use of a high strength filament to secure the membrane 12 allows for a strong clamping force to be achieved with a very low additional thickness - allowing thicknesses on the micrometer scale.

It is possible for the $pCO_2$ sensor 4 to include more than one sensing chamber. For example, two parallel electrodes 10 separated by a longitudinal wall member may be provided on each side of the support 23. A sensing chamber is thereby formed between one electrode 10 on one side of support 23 via the fluid passageways 11 between the ribs 27 on the top of the support 23 to one of the electrodes 10 on the other side of the support 23. A corresponding sensing chamber is provided between the remaining electrodes 10 and the fluid passageways 11 on the bottom of the support 23. An electrode 10 from each of these chambers may be electrically connected to the corresponding electrode from the other chamber, such that the electrical signal from the $pCO_2$ sensor 4 reflects the conductivity of both chambers.

The temperature sensor 5 (not shown in Figures 3 to 8) takes the form of a thermocouple and may be connected to the end of the support 23 or may be provided within the cabling 6. The temperature sensor 5 is used both for $pCO_2$ corrective calculations and for the measured tissue temperatures to be displayed on the monitor 3, which is informative for medical diagnosis. The temperature sensor 5 has a minimum measuring range of at least 33 - 42 °C and a minimum accuracy of +/- 0.2 °C.

The cable 6 is electrically and mechanically connected to the electrodes 10 of the $pCO_2$ sensor 4 and to the temperature sensor 5. The electrodes 10 may be formed as extensions of the conductors of the cable 6. Where the cable 6 and the connection to the support 23 are sufficiently strong, the cable 6 can be used to pull the sensor unit 1 from its position of use. Alternatively, a Kevlar line may be provided, for example incorporated with the cable 6, to provide a strong external mechanical connection.

The membrane 12 may extend proximally from the support 23 with the cable 6 to form a catheter around the cable 6. Alternatively, a separate catheter may be provided for the cable 6. In this case, the separate catheter may be bonded to the support 23 proximally of the electrodes 10 and the membrane 12.

The catheter tip with the integrated $pCO_2$ sensor 4 is placed 0.5 - 4 cm into organ tissue during surgical procedures to monitor ischemia during a period of up to two weeks. The sensor unit 1 may be used in orthopaedic and reconstructive surgery measuring $pCO_2$ in muscle and subcutaneous tissue, and in organs such as the liver, kidneys, pancreas, heart muscle, brain and intestines. An insertion tool (not shown) may be used for the placement of the sensor unit 1, and there may be a fixation aid to keep the sensor tip 24 in position.

The sensor unit 1 has a maximum diameter of 1 mm and the maximum distance from the catheter tip to the sensor element is 2 mm. The $pCO_2$ sensor 4 has a minimum $pCO_2$ measuring range of 2 - 25 kPa, with a minimum detectable $pCO_2$ difference of 0.2 kPa. The response of the $pCO_2$ sensor 4 is less than 20 seconds. The maximum allowable measurement current in any area of the fluid chamber is such that $j < 1$ mA/cm$^2$ while the measuring input voltage is not more than 50 mV RMS.

The electrodes 10 are gold plated and their total area is approximately 3 mm$^2$. The measurement frequency $f_{meas}$ should be higher than 100 Hz. At lower frequencies, polarisation effects in the measurement chamber dominate the measurements. At frequencies above 10 kHz, the low impedance of the capacitances become a significant issue. The measurement resistance $R_{\_measure}$ is in the range of 500 kOhm to 7 MOhm.

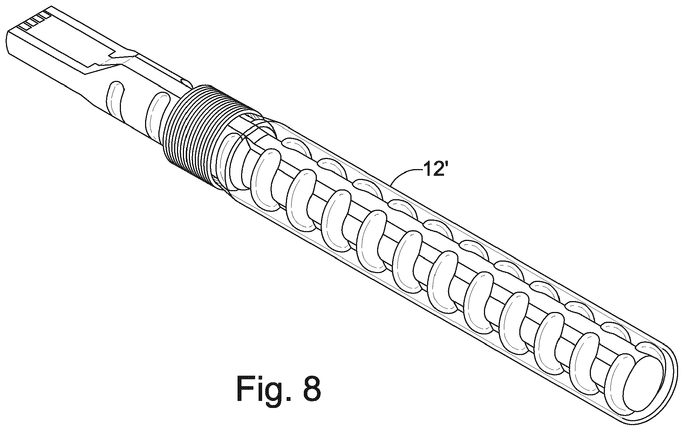

Figure 8 shows an alternative design for the sensor unit 1 having a capsule membrane 12' instead of a tubular membrane 12.

The sensor unit 1 illustrated in Figure 8 operates on the same principle as the sensor unit 1 illustrated in Figures 3 to 7. However, instead of a membrane 12 having an open-ended tubular shape, the membrane 12' has a tubular shape where the distal end is closed. This design means that it is only necessary to secure the membrane to the support 23 at its proximal end. The membrane 12' may be secured using a wound filament 28, in the same manner as discussed above.

Whilst only two designs of the sensor unit 1 have been illustrated, it will be appreciated that many further minor variations in design are possible.

The sensor unit 1 is electrically connected to an electronic surface unit 2 located on the patient skin by the cable 6, which has a length between 5 cm and 2 metre. The maximum diameter of the cable/catheter is 1 mm and the preferred length of the cable/catheter is about 50 cm. The cable/catheter is soft and flexible so that it does not excessively disturb the neighbouring tissue and organs. The cable/catheter and its connections are also sufficiently robust to withstand the strong pulling forces which may be caused by both normal and "abnormal" use.

During sterilisation, storage and transport the sensor unit 1 is covered by deionised, sterile and endotoxin-free water to make sure that there is substantially no net loss of water from the sensor reservoir.

As shown in Figures 1 and 2, the electronic surface unit 2 comprises a sine generator 13 which provides a voltage of at least 5 Volts and a current supply of 50 mV, and is powered via a USB interface 18 or by batteries 14. A filter 15 is provided for filtering or averaging the input of the lock-in amplifier 16. A passive filter can be used which reduces the current consumption. A pre-amplifier 17 is combined with a servo mechanism to remove DC current from the signal to reduce electrolysis effects. According to the servo arrangement, the output of the pre-amplifier is fed back to its input via a low pass filter. Thus, only DC components of the output are fed back and cancel any DC current drawn through the $pCO_2$ sensor 4. In this way, it is ensured that there is no DC current through the $pCO_2$ sensor 4 which would degrade the electrodes 10. The op-amp used in this stage consumes minimal current and has a large CMMR value. At the same time, the bias current is minimal. A lock-in amplifier 16 amplifies the AC signal from the $pCO_2$ sensor 4. This may be built with op-amps or using an IC package with at least 1% accuracy for the signal detection at frequencies lower than 1 kHz. A galvanic division 19 such as an optocoupler or a coil coupler is provided to prevent noise transfer from the monitor unit 3 and associated cabling 18. The optocoupler is normally favoured due to the noise signal ratio. A temperature signal amplification and conditioning unit 20 is provided to amplify the signal from the temperature sensor 5. The electronic unit 2 is powered via the USB interface 18. However, optionally, a rechargeable and changeable standard type battery 14 may be used where the battery capacity is sufficient for at least 14 days continuous monitoring. The surface unit 2 is also provided with an on/off indicator LED 21, and a battery status indicator if appropriate. Communication between the surface unit 2 and the monitor 3 is through the USB interface 18. A USB cable between the surface unit 2 and the monitor 2 is light and flexible and at least 1 m long, preferably about 2 m long.

As shown in Figures 1 and 2, an AC current is generated by sine generator 13 and fed to one of the pCOz sensor electrodes 10 and to a lock-in amplifier 16. The high-pass signal from the other $pCO_2$ electrode 10 is passed through a filter 15 to a low noise amplifier 17 and from there to the lock-in amplifier 16 where it is compared to the reference signal generated by the sine generator 13. Out of phase components, i.e. undesired components, of the signal are rejected and the remaining portion of the signal is amplified. The amplified signal is proportional to $pCO_2$ (or conductance) and is passed on to the monitor 3 for recordal or further manipulation.

The surface unit 2 may also be electrically connected to a reference electrode (not shown) that is electrically connected to the patient's skin. The signal from the reference electrode can be used to compensate the signals from the sensor unit 1 for the effect of electromagnetic noise generated by the patient.

A single surface unit 2 may receive signals from several sensor units 1 and provide a multiplexed output to the monitor unit 3.

The monitor unit 3 comprises a portable PC 7 or similar computing device which can collect signals from a number of different surface units 2 simultaneously. The power supply 22 for the monitor unit 3 is of a medically approved type operating on both 110V and 230V.

The software functions of the monitor unit 3 may be implemented in Labview, a software package available from National Instruments of Austin, Texas and capable of handling up to 4 different surface units simultaneously. The software provides the facility for calibration of the sensor(s) with three calibration points and a second order calibration function. The software can be modified to support any other number of calibration points and type of calibration function. The software also has the facility to smooth the signal from the $pCO_2$ sensor 4 over defined time intervals. It is possible to have at least two alarm levels for the measurement values and two alarm levels for their gradients. The measurement value gradients are calculated for individually defined time intervals. The alarm is both visible and audible. It is possible to stop an alarm indication while keeping the other alarms active. The monitor 3 can log all measured values, parameter settings and alarms throughout a session. With a 30 second logging interval there should be a storage capacity for at least 10 two week sessions on the hard disc. The session log can be saved to a writeable data storage medium in a format readable by Microsoft Excel.

In summary, a physiological sensing device for the measurement of $pCO_2$ includes a closed chamber bounded, at least partially, by a carbon dioxide permeable membrane. There are two electrodes within the chamber. The chamber contains a sensor liquid in contact with electrodes and the membrane. The carbon dioxide permeable membrane is secure to a support structure of the physiological sensing device by a filament wound at least twice around the support structure and on top of the membrane.

**Claims**

1. A physiological sensor (4) for measurement of carbon dioxide, comprising:

   a closed chamber (9, 11) containing a sensor liquid and being bounded, at least partially, by a carbon dioxide permeable membrane (12, 12');
   at least two electrodes (10) provided within the chamber in contact with the sensor liquid;
   a support structure (23) for supporting the membrane (12, 12'); **characterized by**
   at least one filament (28) wound at least twice around the support structure (23) and on top of the membrane (12) for securing the gas-permeable membrane (12, 12') to the support structure (23).

2. A physiological sensor (4) according to claim 1, wherein the at least one filament (28) is formed from one or more of: low-density polyethylene (LDPE), high-density polyethylene (HDPE), ultra-high-molecular-weight polyethylene (UHMWPE), polytetrafluoroethylene (PTFE), ethylene tetrafluoroethylene (ETFE), fluorinated ethylene propylene (FEP), nylon, aramid, or stainless steel.

3. A physiological sensor (4) according to claim 1 or 2, wherein the at least one filament (28) has been secured to one or more of: itself, the support structure (23) and the carbon dioxide permeable membrane (12, 12'), by a fixation medium or by welding of the filament.

4. A physiological sensor (4) according to any preceding claim, wherein the at least one filament (28) comprises a first filament wound around the support structure (23) on a proximal side of the closed chamber (9, 11), and a second filament wound around the support structure (23) on a distal side of the closed chamber (9, 11).

5. A method of securing a carbon dioxide permeable membrane (12, 12') of a physiological sensor (4) for measurement of carbon dioxide, the method comprising:

   applying the carbon dioxide permeable membrane (12, 12') over a support structure (23) so as to define a closed chamber (9, 11) of the physiological sensor (4);
   **characterized by**
   winding at least one filament (28) at least twice around the support structure (23) on top of the membrane (12, 12') so as to secure the membrane (12, 12') to the support structure (23).

6. A method according to claim 5, wherein the at least one filament (28) is formed from one or more of: low-density polyethylene (LDPE), high-density polyethylene (HDPE), ultra-high-molecular-weight polyethylene (UHMWPE), polytetrafluoroethylene (PTFE), ethylene tetrafluoroethylene (ETFE), fluorinated ethylene propylene (FEP), nylon, aramid, or stainless steel.

7. A method according to claim 5 or 6, further comprising:

   securing the at least one filament (28) using a fixation medium to one or more of: itself, the support structure (23) and the carbon dioxide permeable membrane (12, 12'); or
   securing the at least one filament (28) by welding of the at least one filament (28) to one or more of: itself, the support structure (23) and the carbon dioxide permeable membrane (12, 12').

8. A method according to any of claims 5 to 7, wherein the winding comprises:

   winding a first filament around the support structure (23) on a proximal side of the closed chamber (9, 11), and winding a second filament around the support structure (23) on a distal side of the closed chamber (9, 11).

9. A method according to any of claims 5 to 8, wherein the winding is performed whilst the support structure (23) is submerged in a sensor fluid.


**Patentansprüche**

1. Physiologischer Sensor (4) zur Messung von Kohlendioxid, umfassend:

   eine geschlossene Kammer (9, 11), die eine Sensorflüssigkeit enthält und zumindest teilweise durch eine Kohlendioxid-permeable Membran (12, 12') gebunden ist,
   mindestens zwei Elektroden (10), die innerhalb der Kammer in Kontakt mit der Sensorflüssigkeit bereitgestellt sind;
   eine Stützstruktur (23) zum Stützen der Membran (12, 12');
   **gekennzeichnet durch**
   mindestens einen Draht (28), der mindestens zweimal um die Stützstruktur (23) und oben auf der Membran (12) zum Sichern der Gas-permeablen Membran (12, 12') an der Stützstruktur (23) gewickelt ist.

2. Physiologischer Sensor (4) nach Anspruch 1, wobei der mindestens eine Draht (28) aus einem oder mehreren von Folgendem gebildet ist: Polyethylen niedriger Dichte (LDPE), Polyethylen hoher Dichte (HDPE), ultrahochmolekulargewichtigem Polyethylen (UHMWPE), Polytetrafluorethylen (PTFE), Ethylentetrafluorethylen (ETFE), Fluorethylen-Propylen (FEP), Nylon, Aramid oder Edelstahl.

3. Physiologischer Sensor (4) nach Anspruch 1 oder 2, wobei der mindestens eine Draht (28) an einem oder mehreren von Folgendem gesichert wurde: sich selbst, der Stützstruktur (23) und der Kohlendioxid-permeablen Membran (12, 12') durch ein Fixierungsmittel oder durch Schweißen des Drahts.

4. Physiologischer Sensor (4) nach einem der vorhergehenden Ansprüche, wobei der mindestens eine Draht (28) einen ersten Draht, der um die Stützstruktur (23) an einer proximalen Seite der geschlossenen Kammer (9, 11) gewickelt ist, und einen zweiten Draht, der um die Stützstruktur (23) an einer distalen Seite der geschlossenen Kammer (9, 11) gewickelt ist, umfasst.

5. Verfahren zum Sichern einer Kohlendioxid-permeablen Membran (12, 12') eines physiologischen Sensors (4) zur Messung von Kohlendioxid, wobei das Verfahren Folgendes umfasst:

   Aufbringen der Kohlendioxid-permeablen Membran (12, 12') über einer Stützstruktur (23),
   um so eine geschlossene Kammer (9, 11) des physiologischen Sensors (4) zu definieren; **gekennzeichnet durch**
   Wickeln mindestens eines Drahts (28) mindestens zweimal um die Stützstruktur (23) oben auf der Membran (12, 12'), um so die Membran (12, 12') an der Stützstruktur (23) zu sichern.

6. Verfahren nach Anspruch 5, wobei der mindestens eine Draht (28) aus einem oder mehreren von Folgendem gebildet ist: Polyethylen niedriger Dichte (LDPE), Polyethylen hoher Dichte (HDPE), ultrahochmolekulargewichtigem Polyethylen (UHMWPE), Polytetrafluorethylen (PTFE), Ethylentetrafluorethylen (ETFE), Fluorethylen-Propylen (FEP), Nylon, Aramid oder Edelstahl.

7. Verfahren nach Anspruch 5 oder 6, ferner umfassend:

   Sichern des mindestens einen Drahts (28) unter Verwendung eines Fixierungsmittels an einem oder mehreren von Folgendem: sich selbst, der Stützstruktur (23) und der Kohlendioxid-permeablen Membran (12, 12'); oder Sichern des mindestens einen Drahts (28) durch Schweißen des mindestens einen Drahts (28) an einem oder mehreren von Folgendem: sich selbst, der Stützstruktur (23) und der Kohlendioxid-permeablen Membran (12, 12').

**8.** Verfahren nach einem der Ansprüche 5 bis 7, wobei das Wickeln Folgendes umfasst:

Wickeln eines ersten Drahts um die Stützstruktur (23) auf einer proximalen Seite der geschlossenen Kammer (9, 11) und

Wickeln eines zweiten Drahts um die Stützstruktur (23) auf einer distalen Seite der geschlossenen Kammer (9, 11).

**9.** Verfahren nach einem der Ansprüche 5 bis 8, wobei das Wickeln durchgeführt wird, während die Stützstruktur (23) in ein Sensorfluid eingetaucht ist.

**Revendications**

**1.** Capteur physiologique (4) pour la mesure de dioxyde de carbone, comprenant :

une chambre fermée (9, 11) contenant un liquide capteur et étant bordée, au moins partiellement, par une membrane perméable au dioxyde de carbone (12, 12') ;
au moins deux électrodes (10) prévues à l'intérieur de la chambre en contact avec le liquide capteur;
une structure de support (23) pour supporter la membrane (12, 12') ; **caractérisé par**
au moins un filament (28) enroulé au moins deux fois autourde la structure de support (23) et sur le dessus de la membrane (12) pour arrimer la membrane perméable aux gaz (12, 12') à la structure de support (23).

**2.** Capteur physiologique (4) selon la revendication 1, dans lequel l'au moins un filament (28) est formé à partir d'un ou plusieurs composés parmi : polyéthylène basse densité (LDPE), polyéthylène haute densité (HDPE), polyéthylène de poids moléculaire ultra élevée (UHMWPE), polytétrafluoroéthylène (PTFE), éthylène tétrafluoroéthylène (ETFE), éthylène propylène fluoré (FEP), nylon, aramide, ou acier inoxydable.

**3.** Capteur physiologique (4) selon la revendication 1 ou 2, dans lequel le au moins un filament (28) a été arrimé à l'un ou plusieurs parmi : lui-même, la structure de support (23) et la membrane perméable au dioxyde de carbone (12, 12'), par un milieu de fixation ou par soudure du filament.

**4.** Capteur physiologique (4) selon l'une quelconque des revendications précédentes, dans lequel l'au moins un filament (28) comprend un premier filament enroulé autour de la structure de support (23) sur un côté proximal de la chambre fermée (9, 11), et un deuxième filament enroulé autour de la structure de support (23) sur un côté distal de la chambre fermée (9, 11).

**5.** Procédé d'arrimage d'une membrane perméable au dioxyde de carbone (12, 12') d'un capteur physiologique (4) pour la mesure de dioxyde de carbone, le procédé comprenant :

l'application de la membrane perméable au dioxyde de carbone (12, 12') sur une structure de support (23) de manière à définir une chambre fermée (9, 11) du capteur physiologique (4) ;
**caractérisé par**
l'enroulement au moins d'un filament (28) au moins deux fois autour de la structure de support (23) sur le dessus de la membrane (12, 12') de manière à arrimer la membrane (12, 12') à la structure de support (23).

**6.** Procédé selon la revendication 5, dans lequel le au moins un filament (28) est formé à partir d'un ou plusieurs composés suivants : polyéthylène basse densité (LDPE), polyéthylène haute densité (HDPE), polyéthylène de poids moléculaire ultra élevée (UHMWPE), polytétrafluoroéthylène (PTFE), éthylène tétrafluoroéthylène (ETFE), éthylène propylène fluoré (FEP), nylon, aramide, ou acier inoxydable.

**7.** Procédé selon la revendication 5 ou 6, comprenant en outre :

l'arrimage du au moins un filament (28) à l'aide d'un milieu de fixation à un ou plusieurs parmi : lui-même, la structure de support (23) et la membrane perméable au dioxyde de carbone (12, 12') ; ou
l'arrimage du au moins un filament (28) par soudage du au moins un filament (28) à un ou plusieurs parmi : lui-même, la structure de support (23) et la membrane perméable au dioxyde de carbone (12, 12').

**8.** Procédé selon l'une quelconque des revendications 5 à 7, dans lequel l'enroulement comprend :

l'enroulement d'un premier filament autour de la structure de support (23) sur un côté proximal de la chambre fermée (9, 11), et

l'enroulement d'un deuxième filament autour de la structure de support (23) sur un côté distal de la chambre fermée (9, 11).

9. Procédé selon l'une quelconque des revendications 5 à 8, dans lequel l'enroulement est réalisé pendant que la structure de support (23) est immergée dans un fluide capteur.

Fig. 1

Fig. 2

Fig. 3

Fig. 4

EP 4 114 262 B1

Fig. 4a

Fig. 5

23

10

12

28

Fig. 6

10    27    12    28

Fig. 7

Fig. 8

12'

**EP 4 114 262 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 200608505 A
- US 2008319278 A
- US 3659586 A
- US 4005700 A
- WO 2004054438 A
- EP 4045901 A1